(19) Europäisches Patentamt
European Patent Office
Office européen des brevets

(11) **EP 3 145 965 B1**

(12) **EUROPÄISCHE PATENTSCHRIFT**

(45) Veröffentlichungstag und Bekanntmachung des Hinweises auf die Patenterteilung:
**19.02.2020 Patentblatt 2020/08**

(21) Anmeldenummer: **15722719.0**

(22) Anmeldetag: **13.05.2015**

(51) Int Cl.:
*C08F 120/06* (2006.01)      *C07C 51/44* (2006.01)
*C07C 57/04* (2006.01)

(86) Internationale Anmeldenummer:
**PCT/EP2015/060639**

(87) Internationale Veröffentlichungsnummer:
**WO 2015/177029 (26.11.2015 Gazette 2015/47)**

(54) **VERFAHREN ZUR KONTINUIERLICHEN DEHYDRATISIERUNG VON 3-HYDROXYPROPIONSÄURE ZU ACRYLSÄURE**

METHOD FOR THE CONTINUOUS DEHYDRATION OF 3-HYDROXYPROPANOIC ACID TO FORM ACRYLIC ACID

PROCÉDÉ DE DÉSHYDRATATION CONTINUE D'ACIDE 3-HYDROXYPROPIONIQUE POUR FORMER DE L'ACIDE ACRYLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.05.2014 EP 14168770**

(43) Veröffentlichungstag der Anmeldung:
**29.03.2017 Patentblatt 2017/13**

(73) Patentinhaber: **BASF SE**
**67056 Ludwigshafen am Rhein (DE)**

(72) Erfinder:
• **BLASCHKE, Tim**
**2180Z Ekeren (BE)**
• **LANG, Ortmund**
**66909 Quirnbach (DE)**
• **ZAJACZKOWSKI-FISCHER, Marta**
**67141 Neuhofen (DE)**
• **HARTMANN, Marco**
**76751 Jockgrim (DE)**

(74) Vertreter: **BASF IP Association**
**BASF SE**
**G-FLP-C006**
**67056 Ludwigshafen (DE)**

(56) Entgegenhaltungen:
EP-A1- 0 616 998      EP-A1- 2 565 211
WO-A1-2007/106100     WO-A1-2008/023039
WO-A1-2014/111363     WO-A1-2015/036273
DE-A1-102012 212 424

**Beschreibung**

Die Erfindung betrifft ein Verfahren zur Dehydratisierung von wässriger 3-Hydroxypropionsäure zu Acrylsäure, bei dem in einem vorgelagerten Schritt aus wässriger 3-Hydroxypropionsäure ein Teil des Wassers abdestilliert wird, wobei sich ein Teil der monomeren 3-Hydroxypropionsäure unter Wasserabspaltung zu oligomerer 3-Hydroxypropionsäure umsetzt und die so erhaltene Mischung aus monomerer 3-Hydroxypropionsäure und oligomerer 3-Hydroxypropionsäure kontinuierlich dem Sumpf der Destillation entnommen wird, und in einem ersten Schritt eine wässrige Mischung aus 3-Hydroxypropionsäure und oligomerer 3-Hydroxypropionsäure in flüssiger Phase zu Acrylsäure umgesetzt und wässrige Acrylsäure aus der flüssigen Phase abdestilliert wird und in einem zweiten Schritt die wässrige Acrylsäure destillativ in eine acrylsäurereiche Phase und eine wasserreiche Phase aufgetrennt wird.

Acrylsäure ist wegen ihrer sehr reaktiven Doppelbindung sowie ihrer Carbonsäure-Gruppe ein wertvolles Monomer zur Herstellung von Polymeren, z.B. wasserabsorbierende Polymerpartikel, Bindemittel für wässrige Dispersionsfarben und in wässrigem Lösungsmittel dispergierte Klebstoffe.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Acrylsäure wird großtechnisch nahezu ausschließlich aus fossilen Rohstoffen hergestellt. Dies wird von den Konsumenten der Hygieneartikel als nachteilig angesehen. Es besteht daher ein Bedarf die in den Hygieneartikeln eingesetzten wasserabsorbierenden Polymerpartikel aus nachwachsenden Rohstoffen zu erzeugen.

Ein möglicher Weg ist die fermentative Herstellung von 3-Hydroxypropionsäure und deren Umsetzung zu Acrylsäure. Die Herstellung von 3-Hydroxypropionsäure durch Fermentation wird beispielsweise in WO 2012/074818 A2 beschrieben.

Die Dehydratisierung von 3-Hydroxypropionsäure in der Gasphase wird in US 7,538,247 erwähnt.

Die Dehydratisierung von 3-Hydroxypropionsäure in flüssiger Phase wird beispielsweise in WO 2006/092271 A2, WO 2008/023039 A1, JP 2010-180171, EP 2 565 211 A1, WO 2007/106100 A1 und EP 2 565 212 A1 erwähnt.

Aufgabe der vorliegenden Erfindung war die Bereitstellung eines verbesserten Verfahrens zur Herstellung von Acrylsäure auf Basis nachwachsender Rohstoffe.

Gelöst wurde die Aufgabe durch ein Verfahren zur kontinuierlichen Dehydratisierung von wässriger 3-Hydroxypropionsäure zu Acrylsäure, dadurch gekennzeichnet, dass in einem vorgelagerten Schritt aus wässriger 3-Hydroxypropionsäure ein Teil des Wassers abdestilliert wird, wobei sich ein Teil der monomeren 3-Hydroxypropionsäure unter Wasserabspaltung zu oligomerer 3-Hydroxypropionsäure umsetzt und die so erhaltene Mischung aus monomerer 3-Hydroxypropionsäure und oligomerer 3-Hydroxypropionsäure kontinuierlich dem Sumpf der Destillation entnommen wird, in einem ersten Schritt i) eine wässrige Mischung aus 3-Hydroxypropionsäure und oligomerer 3-Hydroxypropionsäure in flüssiger Phase und bei einem Druck von weniger als 900 mbar zu Acrylsäure umgesetzt und wässrige Acrylsäure aus der flüssigen Phase destillativ abgetrennt wird und in einem zweiten Schritt ii) die in Schritt i) erhaltene wässrige Acrylsäure bei einem Druck von weniger als 900 mbar destillativ in eine acrylsäurereiche Phase und eine wasserreiche Phase aufgetrennt wird.

Der Druck ist der Druck im Reaktor bzw. bei einer Destillation der Druck im Destillationssumpf. Die in Schritt i) eingesetzte wässrige Mischung aus monomerer 3-Hydroxypropionsäure und oligomerer 3-Hydroxypropionsäure enthält vorzugsweise von 5 bis 50 Gew.-% Wasser, besonders bevorzugt von 10 bis 40 Gew.-% Wasser, ganz besonders bevorzugt von 15 bis 35 Gew.-% Wasser.

Die in Schritt i) eingesetzte wässrige Mischung aus monomerer 3-Hydroxypropionsäure und oligomerer 3-Hydroxypropionsäure enthält vorzugsweise von 10 bis 60 Gew.-% monomere 3-Hydroxypropionsäure, besonders bevorzugt von 20 bis 50 Gew.-% monomere 3-Hydroxypropionsäure, ganz besonders bevorzugt von 25 bis 45 Gew.-% monomere 3-Hydroxypropionsäure.

Der vorliegenden Erfindung liegt die Erkenntnis zugrunde, dass die Konzentration an Acrylsäure in der flüssigen Phase bei vermindertem Druck niedrig gehalten werden kann. Dadurch wird die Gefahr einer unerwünschten radikalischen Polymerisation in Schritt i) reduziert. Weiterhin entsteht bei der destillativen Auftrennung in Schritt ii) bei vermindertem Druck und den damit verbundenen niedrigeren Temperaturen weniger oligomere Acrylsäure.

Die flüssige Phase in Schritt i) enthält vorteilhaft ein hochsiedendes organisches Lösungsmittel. Das hochsiedende organische Lösungsmittel verdünnt die in der flüssigen Phase gebildete Acrylsäure und hochsiedende Nebenprodukte (Schwersieder).

Die bei der Umsetzung in Schritt i) entstehende wässrige Acrylsäure wird destillativ abgetrennt. Besonders geeignet sind hierfür Rektifikationskolonnen (Rektifikationskolonne 2). Über die Auswahl der Trennstufen und des Rücklaufverhältnisses kann der Gehalt an 3-Hydroxypropionsäure im Destillat niedrig gehalten werden.

Die in Schritt i) erhaltene wässrige Acrylsäure wird destillativ in eine acrylsäurereiche Phase (Rohacrylsäure) und eine wasserreiche Phase (Sauerwasser) aufgetrennt. Zur Erleichterung der Auftrennung kann ein Schleppmittel eingesetzt werden. Geeignete Schleppmittel sind niedrig siedende hydrophobe organische Lösungsmittel. Für die destillative Auftrennung besonders geeignet sind ebenfalls Rektifikationskolonnen (Rektifikationskolonne 3).

Besonders vorteilhaft werden die Rektifikationskolonne 2 und die Rektifikationskolonne 3 zu einer einzigen Rektifikationskolonne 4 zusammengefasst. Dabei wird die Abtrennung der wässrigen Acrylsäure aus der flüssigen Phase in Schritt i) und die Auftrennung der wässrigen Acrylsäure in eine acrylsäurereiche Phase und eine wasserreiche Phase in Schritt ii) in der Rektifikationskolonne 4 durchgeführt, wobei die Abtrennung der wässrigen Acrylsäure aus der flüssigen Phase unterhalb eines Seitenabzugs in der Rektifikationskolonne 4 erfolgt, die Auftrennung der wässrigen Acrylsäure oberhalb des Seitenabzugs erfolgt und die acrylsäurereiche Phase (Rohacrylsäure) am Seitenabzug flüssig entnommen wird.

Die Rohacrylsäure kann durch Kristallisation weiter gereinigt werden.

Oligomere 3-Hydroxypropionsäure ist das Produkt aus mindestens zwei Molekülen 3-Hydroxypropionsäure. Dabei werden die Moleküle untereinander durch Veresterung der Carboxyl-Gruppe des einen Moleküls mit der Hydroxyl-Gruppe des anderen Moleküls miteinander verbunden.

Oligomere Acrylsäure ist das Produkt aus mindestens zwei Molekülen Acrylsäure. Dabei werden die Moleküle untereinander durch Michael-Addition der Carboxyl-Gruppe des einen Moleküls mit der ethylenischen Doppelbindung des anderen Moleküls miteinander verbunden.

Im Folgenden wird das erfindungsgemäße Verfahren beschrieben:

Herstellung der 3-Hydroxypropionsäure

In dem erfindungsgemäßen Verfahren wird vorzugsweise durch Fermentation erzeugte wässrige 3-Hydroxypropionsäure eingesetzt. Ein derartiges Verfahren wird beispielsweise in WO 02/090312 A1 offenbart.

Herstellung von Acrylsäure

Aus der wässrigen 3-Hydroxypropionsäure wird ein Teil des Wassers abdestilliert, wobei sich ein Teil der monomeren 3-Hydroxypropionsäure unter Wasserabspaltung zu oligomerer 3-Hydroxypropionsäure umsetzt.

Die Temperatur bei der Umsetzung beträgt vorzugsweise weniger als 100°C, besonders bevorzugt weniger als 90°C, ganz besonders bevorzugt weniger als 80°C, durchgeführt. Zu hohe Temperaturen begünstigen in Schritt i) die unerwünschte Dehydratisierung von monomerer 3-Hydroxypropionsäure zu Acrylsäure.

Der Druck bei der Umsetzung beträgt vorzugsweise von 10 bis 300 mbar, besonders bevorzugt von 20 bis 200 mbar, ganz besonders bevorzugt von 40 bis 150 mbar. Niedrigere Drücke in Schritt i) ermöglichen eine schonende Entfernung des Wassers aus der flüssigen Phase. Zu niedrige Drücke sind unwirtschaftlich.

Die Wärmezufuhr kann über innen- und/oder außenliegende Wärmetauscher herkömmlicher Bauart und/oder über Doppelwandheizung erfolgen (als Wärmeträger wird vorteilhaft Wasserdampf verwendet). Vorzugsweise erfolgt sie über außenliegende Umlaufverdampfer mit Natur- oder Zwangsumlauf. Besonders bevorzugt werden außenliegende Umlaufverdampfer mit Zwangsumlauf eingesetzt. Derartige Verdampfer werden in EP 0 854 129 A1 beschrieben. Der Einsatz mehrerer Verdampfer, in Reihe oder parallel geschaltet, ist möglich.

Der pH-Wert beträgt während der Destillation vorzugsweise mindestens 1,5, besonders bevorzugt mindestens 1,8, ganz besonders bevorzugt mindestens 2,0. Zu niedrige pH-Werte erhöhen die Oliogomerenbildung und verschieben das Verhältnis von monomerer 3-Hydroxypropionsäure zu oligomerer 3-Hydroxypropionsäure. Ein zu hoher Anteil an langkettigen Oligomeren erhöht die Viskosität und verschlechtert den Wärmeübergang. Es werden also üblicherweise keine die Oligomerisierung katalysierende Verbindungen, insbesondere keine starken Säuren, wie Schwefelsäure, organische Sulfonsäuren und Phosphorsäure, zugesetzt.

Die erhaltene wässrige Mischung aus monomerer 3-Hydroxypropionsäure und oligomerer 3-Hydroxypropionsäure enthält vorzugsweise von 5 bis 50 Gew.-% Wasser, besonders bevorzugt von 10 bis 40 Gew.-% Wasser, ganz besonders bevorzugt von 15 bis 35 Gew.-% Wasser.

Die erhaltene wässrige Mischung aus monomerer 3-Hydroxypropionsäure und oligomerer 3-Hydroxypropionsäure enthält vorzugsweise von 10 bis 60 Gew.-% monomere 3-Hydroxypropionsäure, besonders bevorzugt von 20 bis 50 Gew.-% monomere 3-Hydroxypropionsäure, ganz besonders bevorzugt von 25 bis 45 Gew.-% monomere 3-Hydroxypropionsäure.

Der Wassergehalt wird vorzugsweise um mindestens 5 Gew.-%, besonders bevorzugt um mindestens 10 Gew.-%, ganz besonders bevorzugt um mindestens 15 Gew.-%, gesenkt. Der Wert um den der Wassergehalt gesenkt wurde ist die Differenz aus dem Wassergehalt der eingesetzten wässrigen 3-Hydroxypropionsäure (Edukt) und dem Wassergehalt der erhaltenen wässrigen Mischung aus monomerer 3-Hydroxypropionsäure und oligomerer 3-Hydroxypropionsäure (Produkt).

Der Wassergehalt kann mit den üblichen Methoden bestimmt werden, beispielsweise mittels Karl-Fischer-Titration.

Der Gehalt an monomerer 3-Hydroxypropionsäure wird vorzugsweise um mindestens 5 Gew.-%, besonders bevorzugt um mindestens 15 Gew.-%, ganz besonders bevorzugt um mindestens 25 Gew.-%, gesenkt. Der Wert um den der Gehalt an monomerer 3-Hydroxypropionsäure gesenkt wurde ist die Differenz aus dem Gehalt an monomerer 3-Hydroxypropionsäure der eingesetzten wässrigen 3-Hydroxypropionsäure (Edukt) und dem Gehalt an monomerer 3-Hydroxypropionsäure der erhaltenen wässrigen Mischung aus monomerer 3-Hydroxypropionsäure und oligomerer 3-Hydroxypropionsäure (Produkt).

Der Gehalt an monomerer 3-Hydroxypropionsäure und oligomerer 3-Hydroxypropionsäure kann mittels HPLC bestimmt werden. Zur Bestimmung der oligomeren 3-Hydroxypropionsäure werden die Signale der ersten vier Oligomeren, d.h. bis zum Pentameren, unter Verwendung des Eichfaktors der monomerer 3-Hydroxypropionsäure ausgewertet und die Summe gebildet.

Monomere Acrylsäure und oligomere Acrylsäure lassen sich analog bestimmen.

Das Wasser wird vorteilhaft mittels einer Rektifikationskolonne 1 abgetrennt. Die Rektifikationskolonne 1 ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt.

Der Zulauf in die Rektifikationskolonne 1 erfolgt zweckmäßig in ihren unteren Bereich. Die Zulauftemperatur beträgt vorzugsweise von 20 bis 100°C, besonders bevorzugt von 30 bis 80°C, ganz besonders bevorzugt von 40 bis 60°C. Besonders bevorzugt sind Dual-Flow-Böden unterhalb des Zulaufs (Abtriebsteil) und Thormannböden oberhalb des Zulaufs (Verstärkungsteil). In der Regel sind 2 bis 5 theoretische Böden unterhalb des Zulaufs und 2 bis 15 theoretische Böden oberhalb des Zulaufs der Rektifikationskolonne 1 ausreichend. Die Rektifikation wird üblicherweise so durchgeführt, dass sich der für die Umsetzung geforderte Sumpfdruck einstellt. Der Kopfdruck ergibt sich aus dem Sumpfdruck, der Anzahl und der Art der Kolonneneinbauten sowie den fluiddynamischen Erfordernissen der Rektifikation.

Zur Erhöhung der Verweilzeit und damit zur Steigerung der Umsetzung von monomerer 3-Hydroxypropionsäure zu oligomerer 3-Hydroxypropionsäure ist es vorteilhaft einen Teil der Sumpfflüssigkeit zusammen mit dem Zulauf in den unteren Bereich der Rektifikationskolone 1 zu fördern. Dadurch wird ein Teil der Sumpfflüssigkeit über die Böden unterhalb des Zulaufs (Abtriebsteil) im Kreis gefördert.

Die Rektifikationskolonne 1 ist üblicherweise aus austenitischem Stahl gefertigt, vorzugweise aus dem Werkstoff 1.4571 (nach DIN EN 10020).

Die Abkühlung des am Kopf der Rektifikationskolonne 1 abgetrennten Wassers kann indirekt, beispielsweise durch Wärmetauscher, die dem Fachmann an sich bekannt sind und keiner besonderen Beschränkung unterliegen, oder direkt, beispielsweise durch einen Quench, erfolgen. Dazu wird bereits kondensiertes Wasser mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle des Wassers vorteilhafterweise als Fangboden ausgebildet. Durch Einbauten, die die Durchmischung des gekühlten Wassers mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolone 1 nicht berücksichtigt. Die direkte Kondensation des Wassers kann auch mehrstufig ausgeführt sein, mit nach oben abnehmender Temperatur. Vorzugsweise erfolgt die Abkühlung aber durch indirekte Kühlung.

Die so erhaltene wässrige Mischung aus monomerer 3-Hydroxypropionsäure und oligomerer 3-Hydroxypropionsäure wird kontinuierlich dem Sumpf der Destillation entnommen und zu Acrylsäure umgesetzt.

Die Umsetzung der wässrigen Mischung aus monomerer 3-Hydroxypropionsäure und oligomerer 3-Hydroxypropionsäure zu Acrylsäure wird in flüssiger Phase bei einer Temperatur von vorzugsweise von 130 bis 220°C, besonders bevorzugt von 150 bis 200°C, ganz besonders bevorzugt von 160 bis 190°C, durchgeführt. Der Druck beträgt vorzugsweise von 25 bis 750 mbar, besonders bevorzugt von 50 bis 500 mbar, ganz besonders bevorzugt von 100 bis 300 mbar. Bei niedrigerem Druck enthält die flüssige Phase weniger monomere Acrylsäure und im Falle einer destillativen Abtrennung der entstehenden Acrylsäure wird die unerwünschte Bildung oligomerer Acrylsäure im Kondensat unterdrückt.

Die Wärmezufuhr kann über innen- und/oder außenliegende Wärmetauscher herkömmlicher Bauart und/oder über Doppelwandheizung erfolgen (als Wärmeträger wird vorteilhaft Wasserdampf verwendet). Vorzugsweise erfolgt sie über außenliegende Umlaufverdampfer mit Natur- oder Zwangsumlauf. Besonders bevorzugt werden außenliegende Umlaufverdampfer mit Zwangsumlauf eingesetzt. Derartige Verdampfer werden in EP 0 854 129 A1 beschrieben. Der Einsatz mehrerer Verdampfer, in Reihe oder parallel geschaltet, ist möglich.

Die flüssige Phase enthält vorzugsweise einen Polymerisationsinhibitor 1. Geeignete Polymerisationsinhibitoren 1 sind Phenothiazin, Hydrochinon und/oder Hydrochinonmonomethylether. Ganz besonders bevorzugt sind Phenothiazin und Hydrochinonmonomethylether. Die flüssige Phase enthält vorzugsweise von 0,001 bis 5 Gew.-%, besonders bevorzugt von 0,01 bis 2 Gew.-%, ganz besonders bevorzugt von 0,1 bis 1 Gew.-% des Polymerisationsinhibitors 1. Vorteilhaft wird zusätzlich ein Sauerstoff-haltiges Gas zur Polymerisationsinhibierung eingesetzt. Besonders geeignet sind hierzu Luft/Stickstoff-Gemische mit einem Sauerstoffgehalt von 6 Vol.-% (Magerluft). Wird ein Sauerstoff-haltiges Gas zur Polymerisationsinhibierung eingesetzt, so wird dies vorzugsweise unterhalb des Verdampfers zugeführt.

Die flüssige Phase enthält vorzugsweise von 20 bis 95 Gew.-%, besonders bevorzugt von 40 bis 85 Gew.-%, ganz besonders bevorzugt von 50 bis 80 Gew.-% des hochsiedenden organischen Lösungsmittels.

Der Siedepunkt des hochsiedenden organischen Lösungsmittels liegt bei 1013 mbar im Bereich von vorzugsweise 200 bis 350°C, besonders bevorzugt von 250 bis 320°C, ganz besonders bevorzugt von 280 bis 300°C. Geeignete hochsiedende organische Lösungsmittel sind beispielsweise Dimethylphthalat, Diethylphthalat, Dimethylisophthalat, Diethylisophthalat, Dimethylterephthalat, Diethylterephthalat, Alkansäuren, wie Nonansäure und Dekansäure, Biphenyl und/oder Diphenylether.

Die Umsetzung der wässrigen Mischung aus 3-Hydroxypropionsäure und oligomerer 3-Hydroxypropionsäure kann basisch oder sauer katalysiert werden. Geeignete basische Katalysatoren sind hochsiedende tertiäre Amine, wie Pentamethyldiethylentriamin. Geeignete saure Katalysatoren sind hochsiedende anorganische oder organische Säuren, wie Phosphorsäure und Dodecylbenzolsulfonsäure. Hochsiedend bedeutet hierbei ein Siedepunkt bei 1013 mbar von vorzugsweise mindestens 160°C, besonders bevorzugt mindestens 180°C, ganz besonders bevorzugt mindestens 190°C.

Wird ein Katalysator eingesetzt, so beträgt die Menge an Katalysator in der flüssigen Phase vorzugsweise von 1 bis 60 Gew.-%, besonders bevorzugt von 2 bis 40 Gew.-%, ganz besonders bevorzugt von 5 bis 20 Gew.-%.

Die bei der Umsetzung der wässrigen Mischung aus monomerer 3-Hydroxypropionsäure und oligomerer 3-Hydroxypropionsäure entstehende wässrige Acrylsäure wird vorzugsweise destillativ abgetrennt, besonders bevorzugt mittels einer Rektifikationskolonne (Rektifikationskolonne 2).

Bei Verwendung einer Rektifikationskolonne 2 findet die Umsetzung der wässrigen Mischung aus monomerer 3-Hydroxypropionsäure und oligomerer 3-Hydroxypropionsäure zu Acrylsäure im Sumpf der Rektifikationskolonne 2 statt und die wässrige Mischung aus monomerer 3-Hydroxypropionsäure und oligomerer 3-Hydroxypropionsäure ist der Zulauf der Rektifikationskolonne 2.

Bei Verwendung einer Rektifikationskolonne 2 wird der Polymerisationsinhibitor 1 zumindest teilweise über den Rücklauf dosiert.

Die Rektifikationskolonne 2 ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden.

In der Regel sind 3 bis 10 theoretische Böden in der Rektifikationskolonne 2 ausreichend. Die Rektifikation wird üblicherweise bei vermindertem Druck durchgeführt. Der Kopfdruck beträgt vorzugsweise von 50 bis 900 mbar, besonders bevorzugt von 200 bis 600 mbar, ganz besonders bevorzugt von 300 bis 400 mbar. Bei zu hohem Kopfdruck wird die wässrige Acrylsäure unnötig thermisch belastet und bei zu niedrigem Kopfdruck wird das Verfahren technisch zu aufwendig. Der Sumpfdruck ergibt sich aus dem Kopfdruck, der Anzahl und der Art der Kolonneneinbauten sowie den fluiddynamischen Erfordernissen der Rektifikation.

Die Rektifikationskolonne 2 ist üblicherweise aus austenitischem Stahl gefertigt, vorzugweise aus dem Werkstoff 1.4571 (nach DIN EN 10020).

Die Abkühlung der am Kopf der Rektifikationskolonne 2 abgetrennten wässrigen Acrylsäure kann indirekt, beispielsweise durch Wärmetauscher, die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte wässrige Acrylsäure mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle der wässrigen Acrylsäure vorteilhafterweise als Fangboden ausgebildet. Durch Einbauten, die die Durchmischung der gekühlten wässrigen Acrylsäure mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolonne 2 nicht berücksichtigt. Die direkte Kondensation der wässrigen Acrylsäure kann auch mehrstufig ausgeführt sein, mit nach oben abnehmender Temperatur. Vorzugsweise erfolgt die Abkühlung durch direkte Kühlung.

Ein Teil der am Kopf der Rektifikationskolonne 2 entnommenen wässrigen Acrylsäure, vorzugsweise 10 bis 40 Gew.-% bezogen auf die Gesamtmenge an Destillat, wird als Rücklauf für die Rektifikationskolonne 2 verwendet, der Rest der wässrigen Acrylsäure wird ausgeschleust.

Bei Verwendung eines hochsiedenden organischen Lösungsmittels mit geringer Löslichkeit in Wasser kann das kondensierte Destillat der Rektifikationskolonne 2 mittels eines Phasenscheiders getrennt werden. Die organische Phase kann in die Rektifikationskolonne 2 zurückgeführt werden, beispielsweise in den Sumpf der Rektifikationskolonne 2. Die wässrige Phase kann ebenfalls teilweise in die Rektifikationskolonne 2 zurückgeführt werden, beispielsweise als Rücklauf und zur direkten Kühlung des Brüdens.

Der Sumpfrückstand der Rektifikationskolonne 2 kann ausgeschleust und einer Rückstandsdestillation oder einer Rückstandsspaltung zugeführt werden. Der Sumpfrückstand wird vorzugsweise über einen Feststoffabscheider (Zyklon) geführt und ggf. durch frisches inertes organisches Lösungsmittel 1 ergänzt.

Die erhaltene wässrige Acrylsäure wird destillativ in eine acrylsäurereiche Phase (Rohacrylsäure) und eine wasserreiche Phase (Sauerwasser) aufgetrennt.

Die Wärmezufuhr kann über innen- und/oder außenliegende Wärmetauscher herkömmlicher Bauart und/oder über Doppelwandheizung erfolgen (als Wärmeträger wird vorteilhaft Wasserdampf verwendet). Vorzugsweise erfolgt sie über außenliegende Umlaufverdampfer mit Natur- oder Zwangsumlauf. Besonders bevorzugt werden außenliegende Umlaufverdampfer mit Zwangsumlauf eingesetzt. Derartige Verdampfer werden in EP 0 854 129 A1 beschrieben. Der Einsatz mehrerer Verdampfer, in Reihe oder parallel geschaltet, ist möglich.

Die wässrige Acrylsäure enthält vorzugsweise einen Polymerisationsinhibitor 2. Geeignete Polymerisationsinhibitoren 2 sind Phenothiazin, Hydrochinon und/oder Hydrochinonmonomethylether. Ganz besonders bevorzugt sind Phenothiazin und Hydrochinonmonomethylether. Die flüssige Phase enthält vorzugsweise von 0,001 bis 5 Gew.-%, besonders bevorzugt von 0,01 bis 2 Gew.-%, ganz besonders bevorzugt von 0,1 bis 1 Gew.-% des Polymerisationsinhibitors 2. Vorteilhaft wird zusätzlich ein Sauerstoff-haltiges Gas zur Polymerisationsinhibierung eingesetzt. Besonders geeignet sind hierzu Luft/Stickstoff-Gemische mit einem Sauerstoffgehalt von 6 Vol.-% (Magerluft). Wird ein Sauerstoff-haltiges Gas zur Polymerisationsinhibierung eingesetzt, so wird dies vorzugsweise unterhalb des Verdampfers zugeführt.

Vorteilhaft wird der abgetrennten acrylsäurereichen Phase (Rohacrylsäure) ein Polymerisationsinhibitor 3 zugesetzt. Geeignete Polymerisationsinhibitoren 3 sind Phenothiazin, Hydrochinon und/oder Hydrochinonmonomethylether. Ganz besonders bevorzugt sind Phenothiazin und Hydrochinonmonomethylether.

Zur Unterstützung der Auftrennung der wässrigen Acrylsäure in eine acrylsäurereiche Phase (Rohacrylsäure) und eine wasserreiche Phase (Sauerwasser) kann ein Schleppmittel zugesetzt werden. Geeignete Schleppmittel sind niedrig siedende hydrophobe organische Lösungsmittel mit einer Löslichkeit in Wasser bei 23°C von vorzugsweise weniger als 5 g pro 100 ml Wasser, besonders bevorzugt weniger als 1 g pro 100 ml Wasser, ganz besonders bevorzugt von weniger als 0,2 g pro 100 ml Wasser, und einem Siedepunkt bei 1013 mbar im Bereich von vorzugsweise 60 bis 160°C, besonders bevorzugt von 70 bis 130°C, ganz besonders bevorzugt von 75 bis 115°C. Geeignete hydrophobe organische Lösungsmittel sind beispielsweise aliphatische Kohlenwasserstoffe, wie Hexan, Heptan, Dodecan, Cyclohexan, Methylcyclohexan, Isooctan und hydriertes Triisobutylen, aromatische Kohlenwasserstoffe, wie Benzol, Toluol, Xylol und Ethylbenzol, Ketone, wie Methylisobutylketon, Ether, wie Methyl-tert.-butylether, oder Mischungen daraus.

Zur destillativen Auftrennung der wässrigen Acrylsäure eine acrylsäurereiche Phase (Rohacrylsäure) und eine wasserreiche Phase (Sauerwasser) wird vorzugsweise eine Rektifikationskolonne 3 verwendet.

Bei Verwendung einer Rektifikationskolonne 3 wird der Polymerisationsinhibitor 2 zumindest teilweise über den Rücklauf dosiert.

Die Rektifikationskolonne 3 ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden.

In der Regel sind 10 bis 30 theoretische Böden in der Rektifikationskolonne 3 ausreichend. Die Rektifikation wird üblicherweise bei vermindertem Druck durchgeführt. Der Kopfdruck beträgt vorzugsweise von 50 bis 600 mbar, besonders bevorzugt von 150 bis 400 mbar, ganz besonders bevorzugt von 200 bis 300 mbar. Bei zu hohem Kopfdruck wird die wässrige Acrylsäure unnötig thermisch belastet und bei zu niedrigem Kopfdruck wird das Verfahren technisch zu aufwendig. Der Sumpfdruck ergibt sich aus dem Kopfdruck, der Anzahl und der Art der Kolonneneinbauten sowie den fluidynamischen Erfordernissen der Rektifikation.

Die Rektifikationskolonne 3 ist üblicherweise aus austenitischem Stahl gefertigt, vorzugweise aus dem Werkstoff 1.4571 (nach DIN EN 10020).

Die Abkühlung der am Kopf der Rektifikationskolonne 3 abgetrennten wasserreichen Phase (Sauerwasser) kann indirekt, beispielsweise durch Wärmetauscher, die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte wasserreiche Phase (Sauerwasser) mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle der wasserreichen Phase (Sauerwasser) vorteilhafterweise als Fangboden ausgebildet. Durch Einbauten, die die Durchmischung der gekühlten wasserreichen Phase (Sauerwasser) mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolonne 3 nicht berücksichtigt. Die direkte Kondensation der wasserreichen Phase (Sauerwasser) kann auch mehrstufig ausgeführt sein, mit nach oben abnehmender Temperatur. Vorzugsweise erfolgt die Abkühlung durch direkte Kühlung.

Ein Teil der am Kopf der Rektifikationskolonne 3 kondensierten wasserreichen Phase (Sauerwasser) kann als Rücklauf verwendet werden, der Rest der wasserreichen Phase (Sauerwasser) wird ausgeschleust und zur Rückgewinnung von Acrylsäure einer Sauerwasserextraktion zugeführt werden.

Bei Verwendung eines hydrophoben organischen Lösungsmittels wird das kondensierte Destillat der Rektifikationskolonne 3 mittels eines Phasenscheiders getrennt. Die organische Phase kann in die Rektifikationskolonne 3 zurückgeführt werden, beispielsweise als Rücklauf.

Die dem Sumpf der Rektifikationskolonne 3 entnommene acrylsäurereiche Phase (Rohacrylsäure) kann direkt zur Herstellung wasserabsorbierender Polymerpartikel eingesetzt werden. Vorzugsweise wird die acrylsäurereiche Phase (Rohacrylsäure) durch Kristallisation weiter gereinigt. Die bei der Kristallisation anfallende Mutterlauge kann in die Rektifikationskolonne 3 zurückgeführt werden, vorzugsweise unterhalb der Abnahmestelle für die acrylsäurereiche Phase (Rohacrylsäure).

Die acrylsäurereiche Phase (Rohacrylsäure) kann durch Schichtkristallisation, wie beispielsweise in EP 0 616 998 A1 beschrieben, oder durch Suspensionskristallisation, wie in DE 100 39 025 A1 beschrieben, gereinigt werden. Die Suspensionskristallisation ist bevorzugt. Die Kombination einer Suspensionskristalisation mit einer Waschkolone, wie in WO 2003/041832 A1 beschrieben, ist besonders bevorzugt.

In einer besonders bevorzugten Ausführungsform der vorliegenden Erfindung werden die Abtrennung der wässrigen Acrylsäure aus der flüssigen Phase und die Auftrennung der wässrigen Acrylsäure in eine acrylsäurereiche Phase (Rohacrylsäure) und eine wasserreiche Phase (Sauerwasser) mittels einer Rektifikationskolonne mit einem Seitenabzug (Rektifikationskolonne 4) durchgeführt. Die Rektifikationskolonne 4 vereinigt die Aufgaben der Rektifikationskolonnen 2 und 3 in einer einzigen Rektifikationskolonne. Dabei entspricht der Abschnitt unterhalb des Seitenabzugs der Rektifikationskolonne 2 und der Abschnitt oberhalb des Seitenabzugs der Rektifikationskolonne 3.

Bei Verwendung einer Rektifikationskolonne 4 findet die Umsetzung der wässrigen Mischung aus monomerer 3-Hydroxypropionsäure und oligomerer 3-Hydroxypropionsäure zu Acrylsäure im Sumpf der Rektifikationskolonne 4 statt und die wässrige Mischung aus monomerer 3-Hydroxypropionsäure und oligomerer 3-Hydroxypropionsäure ist der Zulauf der Rektifikationskolonne 4.

Der Zulauf in die Rektifikationskolonne 4 erfolgt zweckmäßig in ihrem unteren Bereich. Vorzugsweise erfolgt er unterhalb des ersten Bodens der Rektifikationskolonne 4. Die Zulauftemperatur beträgt vorzugsweise von 25 bis 150°C, besonders bevorzugt von 40 bis 100°C, ganz besonders bevorzugt von 50 bis 70°C.

Die Wärmezufuhr im Sumpf der Rektifikationskolonne 4 erfolgt über innen- und/oder außenliegende Wärmetauscher (Wärmeträger ist wieder vorzugsweise Wasserdampf) herkömmlicher Bauart und/oder über Doppelwandbeheizung. Vorzugsweise erfolgt sie über außenliegende Umlaufverdampfer mit Natur- oder Zwangsumlauf. Besonders bevorzugt sind außenliegende Umlaufverdampfer mit Zwangsumlauf. Derartige Verdampfer werden in EP 0 854 129 A1 beschrieben. Der Einsatz mehrerer Verdampfer, in Reihe oder parallel geschaltet, ist möglich. Vorzugsweise werden 2 bis 4 Verdampfer parallel betrieben.

Wird ein Sauerstoff-haltiges Gas zur Polymerisationsinhibierung eingesetzt, so wird dies vorzugsweise unterhalb des Verdampfers zugeführt.

Der Sumpfrückstand der Rektifikationskolonne 4 kann ausgeschleust und einer Rückstandsdestillation oder einer Rückstandsspaltung zugeführt werden. Der Sumpfrückstand wird vorzugsweise über einen Feststoffabscheider (Zyklon) geführt und ggf. durch frisches hochsiedendes organisches Lösungsmittel ergänzt.

Über den Seitenabzug der Rektifikationskolonne 4 wird die acrylsäurereiche Phase (Rohacrylsäure) entnommen. Die Entnahme der acrylsäurereichen Phase (Rohacrylsäure) erfolgt auf übliche Weise und unterliegt keiner Beschränkung. Geeignet ist die Entnahme über einen Fangboden, wobei der gesamte Rücklauf aufgefangen wird und ein Teil ausgeschleust und der andere Teil als Rücklauf unterhalb des Fangbodens verwendet wird, oder über einen Boden mit integrierter Abzugsmöglichkeit, vorzugsweise über einen Dual-Flow-Boden mit integrierter Abzugsmöglichkeit.

Die entnommene acrylsäurereiche Phase (Rohacrylsäure) wird mittels eines Wärmetauschers abgekühlt (als Kühlmittel eignen sich z.B. Oberflächenwässer). Der Einsatz mehrerer Wärmetauscher, in Reihe oder parallel geschaltet, ist möglich. Die Wärmetauschern sind dem Fachmann an sich bekannt und unterliegen keiner besonderen Beschränkung.

Die entnommene acrylsäurereiche Phase (Rohacrylsäure) wird ausgeschleust und teilweise als Lösungsmittel für den Polymerisationsinhibitor 2 verwendet.

Die Abkühlung der am Kopf der Rektifikationskolonne 4 abgetrennten wasserreichen Phase (Sauerwasser) kann indirekt, beispielsweise durch Wärmetauscher, die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte wasserreiche Phase (Sauerwasser) mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle der wasserreichen Phase (Sauerwasser) vorteilhafterweise als Fangboden ausgebildet. Durch Einbauten, die die Durchmischung der gekühlten wasserreichen Phase (Sauerwasser) mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolonne 4 nicht berücksichtigt. Die direkte Kondensation der wasserreichen Phase (Sauerwasser) kann auch mehrstufig ausgeführt sein, mit nach oben abnehmender Temperatur. Vorzugsweise erfolgt die Abkühlung durch direkte Kühlung.

Ein Teil der am Kopf der Rektifikationskolonne 4 kondensierten wasserreichen Phase (Sauerwasser) kann als Rücklauf verwendet werden, der Rest der wasserreichen Phase (Sauerwasser) wird ausgeschleust und zur Rückgewinnung von Acrylsäure einer Sauerwasserextraktion zugeführt werden.

Bei Verwendung eines hydrophoben organischen Lösungsmittels wird das kondensierte Destillat der Rektifikationskolonne 4 mittels eines Phasenscheiders getrennt. Die organische Phase kann in die Rektifikationskolonne 4 zurückgeführt werden, beispielsweise als Rücklauf.

In einer bevorzugten Ausführungsform der vorliegenden Erfindung wird eine Trennwandkolonne als Rektifikationskolonne 4 eingesetzt. Eine Trennwandkolonne hat eine vertikale Trennwand, die den Querschnitt eines Teils der Kolonne in zwei Abschnitte aufteilt. Der Rücklauf wird auf die beiden Kolonnenabschnitte verteilt. Der Zulauf und der Seitenabzug der Trennwandkolonne befinden sich auf unterschiedlichen Seiten der Trennwand.

Die der Rektifikationskolonne 4 entnommene Rohacrylsäure kann direkt zur Herstellung wasserabsorbierender Polymerpartikel eingesetzt werden. Vorzugsweise wird die Rohacrylsäure durch Kristallisation weiter gereinigt. Die bei der Kristallisation anfallende Mutterlauge kann in die Rektifikationskolonne 4 zurückgeführt werden, vorzugsweise unterhalb der Abnahmestelle für die Rohacrylsäure.

Die Rohacrylsäure kann durch Schichtkristallisation, wie beispielsweise in EP 0 616 998 A1 beschrieben, oder durch Suspensionskristallisation, wie in DE 100 39 025 A1 beschrieben, gereinigt werden. Die Suspensionskristallisation ist bevorzugt. Die Kombination einer Suspensionskristalisation mit einer Waschkolonne, wie in WO 2003/041832 A1 beschrieben, ist besonders bevorzugt.

Die so hergestellte Acrylsäure kann direkt als Monomer zur Herstellung von Homo- oder Copolymeren, insbesondere Acrylsäure-Homopolymeren, Acrylsäure/Maleinsäureanhydrid-Copolymeren, Acrylsäure/Maleinsäure-Copolymeren und Acrylsäure/Methacrylsäure-Copolymeren, aber auch zur Herstellung von wasserabsorbierenden Polymerpartikeln und Acrylsäureestern, z.B. Methylacrylat, Ethylacrylat, n-Butylacrylat und 2-Ethylhexylacrylat, verwendet werden.

Herstellung wasserabsorbierender Polymerpartikel

Wasserabsorbierender Polymerpartikel werden durch Polymerisation einer Monomerlösung oder -suspension, enthaltend

a) mindestens ein ethylenisch ungesättigtes, säuregruppentragendes Monomer, das zumindest teilweise neutralisiert sein kann, insbesondere teilneutralisierte Acrylsäure
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Weitere geeignete Monomere a) sind beispielsweise ethylenisch ungesättigte Sulfonsäuren, wie Styrolsulfonsäure und 2-Acrylamido-2-methylpropansulfonsäure (AMPS).

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,5 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die in Acrylsäure üblicherweise eingesetzten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2, WO 2008/052971 A1 und WO 2011/026876 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 5 Gew.-%, besonders bevorzugt 0,02 bis 2 Gew.-%, ganz besonders bevorzugt 0,05 bis 1 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1 Gew.-%, vorzugsweise 0,005 bis 0,5 Gew.-%, besonders bevorzugt 0,02 bis 0,2 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%, jeweils bezogen auf die wasserabsorbierenden Polymerpartikel. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Methoden

Bestimmung des Gehalts an 3-Hydroxoypropionsäure und Acrylsäure

Die Gehalte an 3-Hydroxoypropionsäure und Acrylsäure werden durch Umkehrphasenchromatographie mit Ultraviolett-Detektion bestimmt.

Zu Probenvorbereitung werden ca. 100 bis 300 mg Probe in einen 50ml Messkolben eigewogen und mit Eluent A aufgefüllt. Eluent A ist eine Mischung aus 1000 ml Wasser und 1 ml 0,5molarer Schwefelsäure.

Zur Kalibrierung der 3-Hydroxypropionäure werden vier Einwaagen (ca. 280 mg, 180 mg, 90 mg und 60 mg) verwendet, wobei vor dem Auffüllen des 50ml Messkolbens mit ca. 100 $\mu$l 25gew.-%iger Schwefelsäure auf einen pH-Wert von 3 bis 4 angesäuert wird (ggf. nachsäuern). Der Kalibrierbereich beträgt 0,1 bis 280 mg/50ml.

Zur Kalibrierung der Acrylsäure werden mindestens zwei Einwaagen auf mindestens sechs Konzentrationen verdünnt. Der Kalibrierbereich beträgt 0,01 bis 0,9 mg/50ml.

Zur Umkehrphasenchromatographie wird eine Trennsäule vom Typ Prontosil 120-3-C18 AQ 3$\mu$m, 150x4,6mm (BISCHOFF Analysentechnik und -geräte GmbH, Leonberg, Deutschland) verwendet. Die Temperatur beträgt 25°C das Injektionsvolumen beträgt 50 $\mu$l, der Durchfluss beträgt 1,5 ml/min und die Laufzeit beträgt 15 Minuten. Der UV-Detektor wird auf 205 nm eingestellt. Von Anfang bis 8 Minuten wird 100 Gew.-% Eluent A, von 8 bis 11,5 Minuten wird eine Mischung aus 40 Gew.-% Eluent A und 60 Gew.-% Eluent B, von 11,5 Minuten bis Ende wird 100 Gew.-% Eluent A verwendet. Eluent B ist Acetonitril.

Bestimmung des Gehalts an oligomerer 3-Hydroxoypropionsäure und oligomerer Acrylsäure

Die Gehalte an oligomerer 3-Hydroxoypropionsäure und oligomerer Acrylsäure werden durch Ionenausschluss-schromatographie mit Brechungsindex-Detektion bestimmt.

Zur Probenvorbereitung werden die zu analysierenden Komponenten mittels einer Festphasenextraktion (solid phase extraction) von der Probenmatrix getrennt. Dazu wird eine SPE-Kartusche vom Typ Bakerband SiOH 6 ml, 1000 mg (J.T.Baker, Avantor Performance Materials, Inc., Center Valley, PA, USA) verwendet. Die SPE-Kartusche wird mit 6 ml Methanol aktiviert und zweimal mit je 6 ml Eluent gespült. Die SPE-Kartusche darf nie trockenlaufen. Anschließend wird die Probe auf die SPE-Kartusche pipettiert und zehnmal mit je 1 ml Eluent in einen 10 ml Messkolben gespült. Die eingesetzte Probenmenge beträgt bei Sumpfproben 65 $\mu$l, bei Kopfproben 85 $\mu$l und bei Extraktproben 75 $\mu$l. Sofern die Proben kein hydrophobes Lösungsmittel (hochsiedendes organisches Lösungsmittel, Schleppmittel) enthalten, können diese Proben ohne Extraktion aufgespritzt werden, dazu werden 85 $\mu$l direkt in 10 ml Eluent gelöst. Als Eluent wird 0,1 Vol.-%ige wässrige Phosphorsäure verwendet.

Zur Ionenausschlusschromatographie werden zwei Trennsäulen vom Typ Shodex RSpak KC-811, 300x8mm (SHOWA DENKO K.K. Shodex (Separation & HPLC) Group, Kawasaki, Japan) hintereinander geschaltet verwendet. Die Temperatur beträgt 40°C, das Injektionsvolumen beträgt 100 $\mu$l, der Durchfluss beträgt 1,0 ml/min und die Laufzeit beträgt 45 Minuten. Als Eluent wird 0,1 gew.-%ige wässriger Phosphorsäure verwendet. Der Autosampler wird auf 15°C gekühlt.

Zur Auswertung wird vor dem Integrieren ein Blindwertabzug gemacht. Dazu wird Eluent injiziert und das so erhaltene Chromatogramm vom Probenchromatogramm abgezogen. Die Auswertung erfolgt über Flächenprozent, wobei mittels folgender Formel in Gewichtsprozent umgerechnet wird:

$$Gewichts\%(Oligomer) = \frac{Gewichts\%(Monomer)}{Fl\ddot{a}chen\%(Monomer)} xFl\ddot{a}chen\%(Oligomer)$$

Zur Auswertung der Oligomeren werden jeweils die Gehalte der Dimeren, Trimeren, Tetrameren und Pentameren (d.h. n = 2 bis 5) addiert. Die Retentionszeiten werden durch Injektion von 3-Hydroxypropionsäure und Diacrylsäure kontrolliert.

Bestimmung des pH-Werts

Zur Bestimmung des pH-Werts werden 1,0 g Probe in 10 ml entmineralisiertem Wasser bei 25°C gelöst bzw. suspendiert. Nach 10 Minuten wird der pH-Wert der wässrigen Phase bei 25°C mittels einer pH-Elektrode gemessen.

Beispiele

Beispiel 1 (Referenzbeispiel)

In einem 2l Dreihalskolben mit Doppelmantel und Destillationsaufsatz wurden 1.500 g einer ca. 30 gew.-%igen wässrigen 3-Hydroxypropionsäure vorgelegt, 3 Stunden bei 100 mbar Wasser abdestilliert und der verbliebene Rückstand bei 40 mbar destilliert. Der Doppelmantel wurde mittels Wärmeträgeröls erwärmt. Der pH-Wert lag während der Destillation im Bereich von 2 bis 3. Die Zusammensetzung von Destillat und Destillationsrückstand wurde analysiert.

Tab. 1: Zusammensetzung des Destillats

| Zeit [h] | 3HPA [Gew.-%] | AS [Gew.-%] | Oligo-3HPA [Gew.-%] | Oligo-AS [Gew.-%] |
|---|---|---|---|---|
| 2,9 | 36,5 | 9,3 | 2,1 | 0,0 |
| 5,5 | 38,2 | 19,4 | 6,3 | 1,6 |
| 6,7 | 12,1 | 51,5 | 6,0 | 20,2 |
| 7,6 | 2,2 | 62,1 | 5,8 | 26,8 |
| 8,8 | 0,5 | 57,6 | 0,2 | 41,4 |

Tab. 2: Zusammensetzung des Destillationsrückstands

| Zeit [h] | Temperatur [°C] | 3HPA [Gew.-%] | AS [Gew.-%] | Oligo-3HPA [Gew.-%] | Oligo-AS [Gew.-%] |
|---|---|---|---|---|---|
| 2,9 | 154 | 42,3 | 0,2 | 41,5 | 9,3 |
| 5,5 | 180 | 16,3 | 0,2 | 55,9 | 21,5 |
| 6,7 | 221 | 1,7 | 0,3 | 56,9 | 37,7 |
| 7,6 | 224 | 0,3 | 0,3 | 38,0 | 59,7 |
| 8,8 | 230 | 0,1 | 0,4 | 31,2 | 58,1 |

3HPA 3-Hydroxypropionsäure
AS Acrylsäure
Oligo-3HPA oligomere 3-Hydroxypropionsäure
Oligo-AS oligomere Acrylsäure

Die Ergebnisse zeigen, dass im Destillationsrückstand zunächst monomere 3-Hydroxypropionsäure in oligomere 3-Hydroxypropionsäure umgewandelt wird. Erst danach entsteht nennenswert Acrylsäure und oligomere Acrylsäure. Vermutlich läuft die Dehydratisierung von 3-Hydroxypropionsäure über oligomere 3-Hydroxypropionsäure als Zwischenstufe. Für eine hohe Ausbeute und eine hohe Selektivität muss daher monomere 3-Hydroxypropionsäure in oligomere 3-Hydroxypropionsäure umgewandelt werden.

Beispiel 2

Die Umsetzung von monomerer 3-Hydroxypropionsäure zu oligomerer 3-Hydroxypropionsäure wird in einem Reaktor mit Zwangsumlaufentspannungsverdampfer und aufgesetzter Rektifikationskolonne 1 durchgeführt.

Als Reaktor wird ein 3l Glasbehälter mit Doppelmantel eingesetzt. Die Flüssigkeitsmenge im Reaktor beträgt ca. 2500 g. Der Reaktor ist gleichzeitig der Sumpf der Rektifikationskolonne 1.

Der Zwangsumlaufentspannungsverdampfer besteht aus einer Pumpe, einem Wärmetauscher und einem Druckhalteventil. Der Reaktorinhalt wird mittels der Pumpe über den Wärmetauscher und das Druckhalteventil im Kreis gefördert.

Als Rektifikationskolonne 1 wird eine 10bödige Glockenbodenkolone mit einem Innendurchmesser von 50 mm eingesetzt. Die Rektifikationskolonne 1 wird elektrisch begleitbeheizt.

Als Zulauf werden 1000 g/h wässrige 3-Hydroxypropionsäure auf den 5. Boden der Glockenbodenkolone gefördert. Die wässrige 3-Hydroxypropionsäure hat die folgende Zusammensetzung:

40,2 Gew.-% Wasser,
2,1 Gew.-% Acrylsäure,
0,1 Gew.-% oligomere Acrylsäure,
52,6 Gew.-% 3-Hydroxypropionsäure und
5,0 Gew.-% oligomere 3-Hydroxypropionsäure

Durch den Zwangsumlaufentspannungsverdampfer wird der Reaktorinhalt im Kreis gefördert. Vor dem Druckhalteventil beträgt der Druck 2,0 bar und die Temperatur 175°C.

Der Druck am Kopf der Rektifikationskolonne 1 beträgt 100 mbar. Der Brüden wird mittels eines Kühlers kondensiert und teilweise als Rücklauf in die Rektifikationskolonne 1 zurückgeführt und teilweise ausgeschleust. Es werden 298,8 g/h Kondensat ausgeschleust. Das Kondensat hat die folgende Zusammensetzung:

99,1 Gew.-% Wasser,
0,9 Gew.-% Acrylsäure und
<0,0001 Gew.-% 3-Hydroxypropionsäure

Aus dem Reaktor werden 701,2 g/h einer wässrigen Mischung von monomerer 3-Hydroxypropionsäure und oligomerer 3-Hydroxypropionsäure ausgeschleust. Die Mischung hat die folgende Zusammensetzung:

20,1 Gew.-% Wasser,
0,2 Gew.-% Acrylsäure,
2,5 Gew.-% oligomere Acrylsäure,
32,4 Gew.-% 3-Hydroxypropionsäure und
44,8 Gew.-% oligomere 3-Hydroxypropionsäure

Beispiel 3

Die Umsetzung von monomerer 3-Hydroxypropionsäure zu oligomerer 3-Hydroxypropionsäure wird in einem Reaktor mit Zwangsumlaufentspannungsverdampfer und aufgesetzter Rektifikationskolonne 1 durchgeführt.
Als Reaktor wird ein 3l Glasbehälter mit Doppelmantel eingesetzt. Die Flüssigkeitsmenge im Reaktor beträgt ca. 2500 g. Der Reaktor ist gleichzeitig der Sumpf der Rektifikationskolonne 1.
Der Zwangsumlaufentspannungsverdampfer besteht aus einer Pumpe, einem Wärmetauscher und einem Druckhalteventil. Der Reaktorinhalt wird mittels der Pumpe über den Wärmetauscher und das Druckhalteventil im Kreis gefördert.
Als Rektifikationskolonne 1 wird eine 10bödige Glockenbodenkolone mit einem Innendurchmesser von 50 mm eingesetzt. Die Rektifikationskolonne 1 wird elektrisch begleitbeheizt.
Als Zulauf werden 1000 g/h wässrige 3-Hydroxypropionsäure auf den 5. Boden der Glockenbodenkolone gefördert. Die wässrige 3-Hydroxypropionsäure hat die folgende Zusammensetzung:

40,2 Gew.-% Wasser,
2,1 Gew.-% Acrylsäure,
0,1 Gew.-% oligomere Acrylsäure,
52,6 Gew.-% 3-Hydroxypropionsäure und
5,0 Gew.-% oligomere 3-Hydroxypropionsäure

Durch den Zwangsumlaufentspannungsverdampfer wird der Reaktorinhalt im Kreis gefördert. Vor dem Druckhalteventil beträgt der Druck 2,0 bar und die Temperatur 175°C.
Es werden 500 g/h des Reaktorinhalts entnommen, mit dem Zulauf gemischt und auf den 5. Boden der Glockenbodenkolone gefördert.
Der Druck am Kopf der Rektifikationskolonne 1 beträgt 100 mbar. Der Brüden wird mittels eines Kühlers kondensiert und teilweise als Rücklauf in die Rektifikationskolonne 1 zurückgeführt und teilweise ausgeschleust. Es werden 314,9 g/h Kondensat ausgeschleust. Das Kondensat hat die folgende Zusammensetzung:

99,35 Gew.-% Wasser,
0,65 Gew.-% Acrylsäure und
<0,0001 Gew.-% 3-Hydroxypropionsäure

Aus dem Reaktor werden 685,1 g/h einer wässrigen Mischung aus monomerer 3-Hydroxypropionsäure und oligomerer 3-Hydroxypropionsäure ausgeschleust. Die Mischung hat die folgende Zusammensetzung:

18,7 Gew.-% Wasser,
<0,05 Gew.-% Acrylsäure,
2,9 Gew.-% oligomere Acrylsäure,
28,4 Gew.-% 3-Hydroxypropionsäure und
50,0 Gew.-% oligomere 3-Hydroxypropionsäure

Beispiel 4

Die Umsetzung von einer wässrigen Mischung aus monomerer 3-Hydroxypropionsäure und oligomerer 3-Hydroxypropionsäure zu Acrylsäure wird in einem Reaktor mit Zwangsumlaufentspannungsverdampfer und aufgesetzter Rektifikationskolonne 4 durchgeführt.

Als Reaktor wird ein 5l Glasbehälter mit Doppelmantel eingesetzt. Die Flüssigkeitsmenge im Reaktor beträgt ca. 4000 g. Die Temperatur in Reaktor beträgt 174°C. Der Druck im Reaktor beträgt 360 mbar. Der Reaktor ist gleichzeitig der Sumpf der Rektifikationskolonne 4.

Der Zwangsumlaufentspannungsverdampfer besteht aus einer Pumpe, einem Wärmetauscher und einem Druckhalteventil. Der Reaktorinhalt wird mittels der Pumpe über den Wärmetauscher und das Druckhalteventil im Kreis gefördert. Der Zulauf an wässriger Mischung aus monomerer 3-Hydroxypropionsäure und oligomerer 3-Hydroxypropionsäure und an hochsiedendem Lösungsmittel wird vor dem Wärmetauscher in den Kreis dosiert. Unterhalb des Wärmetauschers werden 9 l/h eines Luft/Stickstoff-Gemisches mit einem Sauerstoffgehalt von 6 Vol.-% (Magerluft) in den Kreis dosiert.

Es werden 250 g/h wässrige Mischung aus monomerer 3-Hydroxypropionsäure und oligomerer 3-Hydroxypropionsäure und 35 g/h hochsiedendes Lösungsmittel als Zulauf eingesetzt. Die wässrige Mischung aus monomerer 3-Hydroxypropionsäure und oligomerer 3-Hydroxypropionsäure hat folgende Zusammensetzung:

29,5 Gew.-% Wasser,

0,7 Gew.-% Acrylsäure,

0,1 Gew.-% oligomere Acrylsäure

39,8 Gew.-% 3-Hydroxypropionsäure.

27,3 Gew.-% oligomere 3-Hydroxypropionsäure,

0,9 Gew.-% 2-Hydroxypropionsäure,

0,4 Gew.-% Bernsteinsäure,

0,0015 Gew.-% Formaldehyd,

0,001 Gew.-% Acetaldehyd,

0,012 Gew.-% Arabitol,

0,041 Gew.-% Erythritol,

0,7 Gew.-% 2-Hydroxy(iso)valeriansäure,

Glukose, Glukoseester und weitere Schwersieder

Als hochsiedendes Lösungsmittel wurde Diethylphthalat eingesetzt. Das hochsiedende Lösungsmittel enthielt zusätzlich 0,1 Gew.-% Phenothiazin und 0,5 Gew.-% Hydrochinonmonomethylether.

Dem Reaktor werden 54 g/h Rückstand entnommen.

Die Rektifikationskolonne 4 hat einen Innendurchmesser von 50 mm und wird elektrisch begleitbeheizt. Die Rektifikationskolonne hat insgesamt 80 Böden und einen Seitenabzug, wobei sich unterhalb des Seitenabzugs 20 Dual-Flow-Böden und oberhalb des Seitenabzugs 60 Glockenböden befinden.

Der Seitenabzug zwischen dem 20. Und 21. Boden ist ein Fangboden. Dort wird die Flüssigkeit vollständig als Rohacrylsäure entnommen und mittels einer Pumpe durch einen Wärmetauscher gefördert, dabei auf 15°C gekühlt und in eine Suspensionskristallisation überführt. Es werden 294 g/h Rohacrylsäure mit folgender Zusammensetzung erhalten:

3,0 Gew.-% Wasser,

97,0 Gew.-% Acrylsäure,

<0,001 Gew.-% 3-Hydroxypropionsäure.

<0,001 Gew.-% Diethylphthalat,

0,020 Gew.-% Phenothiazin

0,040 Gew.-% Hydrochinonmonomethylether

<0,001 Gew.-% Ethanol,

0,006 Gew.-% Ethylacrylat,

<0,001 Gew.-% 2-Hydroxypropionsäure,

<0,001 Gew.-% Formaldehyd,

<0,001 Gew.-% Acetaldehyd und

<0,001 Gew.-% 2-Hydroxy(iso)valeriansäure

Zur Suspensionskristallisation wird ein 3l Glasbehälter mit Wendelrührer verwendet. Die Kristallisationswärme wird über einen gekühlten Doppelmantel abgeführt. Die Temperatur der Kristallsuspension im Glasbehälter beträgt 7,9°C. Die bei der Kristallisation erzeugte Kristallsuspension wird mittels einer Zentrifuge bei 2000 U/min und einer Schleuderzeit von 1 min diskontinuierlich in Kristalle und Mutterlauge getrennt. Die Kristalle werden mit Reinacrylsäure gewaschen und aufgeschmolzen. Die Mutterlauge wird auf 70°C erwärmt und vollständig auf den 10. Boden der Rektifikationskolonne 4 dosiert.

Als Puffer zwischen der kontinuierlichen Rektifikation und der diskontinuierlichen Suspensionskristallisation werden zwei 5l Glasbehälter mit Doppelmantel eingesetzt.

Auf den 45. Boden der Rektifikationskolonne 4 werden 10 g/h einer 0,5 gew.-%igen Lösung von Phenothiazin in Rohacrylsäure dosiert und auf den 80. Boden der Rektifikationskolonne 4 werden 5 g/h einer 1,0 gew.-%igen Lösung von Hydrochinonmonomethylether in Sauerwasser dosiert.

Der am Kopf der Rektifikationskolonne 4 anfallende Brüden wird in einem Wärmetauscher auf 20°C gekühlt und kondensiert (Sauerwasser). Von dem Sauerwasser werden 7000 g/h vor dem Wärmetauscher im Brüden versprüht, 184 g/h auf den 80. Boden der Rektifikationskolonne 4 dosiert und 83 g/h ausgeschleust. Das Sauerwasser hat die folgende Zusammensetzung:

95,0 Gew.-% Wasser,
5,0 Gew.-% Acrylsäure,
0,025 Gew.-% Ethanol,
0,015 Gew.-% Ethylacrylat,
<0,001 Gew.-% Formaldehyd und
<0,001 Gew.-% Acetaldehyd

Das Abgas wird über einen weiteren Wärmetauscher (Nachkühler) geführt.

Beispiel 5

Die Umsetzung von einer wässrigen Mischung aus monomerer 3-Hydroxypropionsäure und oligomerer 3-Hydroxypropionsäure zu Acrylsäure wird in einem Reaktor mit Zwangsumlaufentspannungsverdampfer und aufgesetzter Rektifikationskolonne 4 durchgeführt. Am Kopf der Rektifikationskolonne wird ein Schleppmittel zugesetzt.

Als Reaktor wird ein 5l Glasbehälter mit Doppelmantel eingesetzt. Die Flüssigkeitsmenge im Reaktor beträgt ca. 4000 g. Die Temperatur in Reaktor beträgt 167°C. Der Druck im Reaktor beträgt 230 mbar. Der Reaktor ist gleichzeitig der Sumpf der Rektifikationskolonne 4.

Der Zwangsumlaufentspannungsverdampfer besteht aus einer Pumpe, einem Wärmetauscher und einem Druckhalteventil. Der Reaktorinhalt wird mittels der Pumpe über den Wärmetauscher und das Druckhalteventil im Kreis gefördert. Der Zulauf an wässriger Mischung aus monomerer 3-Hydroxypropionsäure und oligomerer 3-Hydroxypropionsäure und an hochsiedendem Lösungsmittel wird vor dem Wärmetauscher in den Kreis dosiert. Unterhalb des Wärmetauschers werden 13 l/h eines Luft/Stickstoff-Gemisches mit einem Sauerstoffgehalt von 6 Vol.-% (Magerluft) in den Kreis dosiert.

Es werden 250 g/h wässrige Mischung aus monomerer 3-Hydroxypropionsäure und oligomerer 3-Hydroxypropionsäure und 35 g/h hochsiedendes Lösungsmittel als Zulauf eingesetzt. Die wässrige Mischung aus monomerer 3-Hydroxypropionsäure und oligomerer 3-Hydroxypropionsäure hat folgende Zusammensetzung:

29,5 Gew.-% Wasser,
0,7 Gew.-% Acrylsäure,
0,1 Gew.-% oligomere Acrylsäure
39,8 Gew.-% 3-Hydroxypropionsäure.
27,3 Gew.-% oligomere 3-Hydroxypropionsäure,
0,9 Gew.-% 2-Hydroxypropionsäure,
0,4 Gew.-% Bernsteinsäure,
0,0015 Gew.-% Formaldehyd,
0,001 Gew.-% Acetaldehyd,
0,012 Gew.-% Arabitol,
0,041 Gew.-% Erythritol,
0,7 Gew.-% 2-Hydroxy(iso)valeriansäure,
Glukose, Glukoseester und weitere Schwersieder

Als hochsiedendes Lösungsmittel wurde Diethylphthalat eingesetzt. Das hochsiedende Lösungsmittel enthielt zusätzlich 0,1 Gew.-% Phenothiazin und 0,5 Gew.-% Hydrochinonmonomethylether.

Dem Reaktor werden 53 g/h Rückstand entnommen.

Die Rektifikationskolonne 4 hat einen Innendurchmesser von 50 mm und wird elektrisch begleitbeheizt. Die Rektifikationskolonne hat insgesamt 60 Böden und einen Seitenabzug, wobei sich unterhalb des Seitenabzugs 20 Dual-Flow-Böden und oberhalb des Seitenabzugs 40 Glockenböden befinden.

Der Seitenabzug zwischen dem 20. Und 21. Boden ist ein Fangboden. Dort wird die Flüssigkeit vollständig als Rohacrylsäure entnommen und mittels einer Pumpe durch einen Wärmetauscher gefördert, dabei auf 15°C gekühlt und in eine Suspensionskristallisation überführt. Es werden 304 g/h Rohacrylsäure mit folgender Zusammensetzung erhalten:

3,7 Gew.-% Wasser,
96,3 Gew.-% Acrylsäure,
<0,001 Gew.-% 3-Hydroxypropionsäure.
<0,001 Gew.-% Diethylphthalat,
0,020 Gew.-% Phenothiazin
0,040 Gew.-% Hydrochinonmonomethylether
<0,001 Gew.-% Ethanol,
<0,001 Gew.-% Ethylacrylat,
<0,001 Gew.-% 2-Hydroxypropionsäure,
<0,001 Gew.-% Formaldehyd,
<0,001 Gew.-% Acetaldehyd,
<0,001 Gew.-% 2-Hydroxy(iso)valeriansäure und
<0,001 Gew.-% Toluol

Zur Suspensionskristallisation wird ein 3l Glasbehälter mit Wendelrührer verwendet. Die Kristallisationswärme wird über einen gekühlten Doppelmantel abgeführt. Die Temperatur der Kristallsuspension im Glasbehälter beträgt 7,9°C. Die bei der Kristallisation erzeugte Kristallsuspension wird mittels einer Zentrifuge bei 2000 U/min und einer Schleuderzeit von 1 min diskontinuierlich in Kristalle und Mutterlauge getrennt. Die Kristalle werden mit Reinacrylsäure gewaschen und aufgeschmolzen. Die Mutterlauge wird auf 70°C erwärmt und vollständig auf den 10. Boden der Rektifikationskolonne 4 dosiert.

Als Puffer zwischen der kontinuierlichen Rektifikation und der diskontinuierlichen Suspensionskristallisation werden zwei 5l Glasbehälter mit Doppelmantel eingesetzt.

Auf den 45. Boden der Rektifikationskolonne 4 werden 10 g/h einer 0,5 gew.-%igen Lösung von Phenothiazin in Rohacrylsäure dosiert und auf den 80. Boden der Rektifikationskolonne 4 werden 5 g/h einer 1,0 gew.-%igen Lösung von Hydrochinonmonomethylether in Sauerwasser dosiert.

Der am Kopf der Rektifikationskolonne 4 anfallende Brüden wird in einem Wärmetauscher auf 20°C gekühlt, kondensiert und in einem Phasenscheider gesammelt. Als Schleppmittel wird Toluol eingesetzt. Es werden 5 g/h Toluol in die organische Phase dosiert um Verluste an Schleppmittel auszugleichen.

Von der organischen Phase werden 7000 g/h vor dem Wärmetauscher im Brüden versprüht und 465 g/h auf den 30. Boden der Rektifikationskolonne 4 dosiert. Die wässrige Phase (Sauerwasser) wird ausgeschleust. Das Sauerwasser hat die folgende Zusammensetzung:

99,9 Gew.-% Wasser,
0,022 Gew.-% Acrylsäure,
0,014 Gew.-% Ethanol,
0,0030 Gew.-% Ethylacrylat,
0,0013 Gew.-% Formaldehyd,
<0,001 Gew.-% Acetaldehyd und
0,068 Gew.-% Toluol

Das Abgas wird über einen weiteren Wärmetauscher (Nachkühler) geführt.

## Patentansprüche

1. Verfahren zur kontinuierlichen Dehydratisierung von wässriger 3-Hydroxypropionsäure zu Acrylsäure, **dadurch gekennzeichnet, dass** in einem vorgelagerten Schritt aus wässriger 3-Hydroxypropionsäure ein Teil des Wassers abdestilliert wird, wobei sich ein Teil der monomeren 3-Hydroxypropionsäure unter Wasserabspaltung zu oligomerer 3-Hydroxypropionsäure umsetzt und die so erhaltene Mischung aus monomerer 3-Hydroxypropionsäure und oligomerer 3-Hydroxypropionsäure kontinuierlich dem Sumpf der Destillation entnommen wird, in einem ersten Schritt i) eine wässrige Mischung aus 3-Hydroxypropionsäure und oligomerer 3-Hydroxypropionsäure in flüssiger Phase und bei einem Druck von weniger als 900 mbar zu Acrylsäure umgesetzt und wässrige Acrylsäure aus der flüssigen Phase destillativ abgetrennt wird und in einem zweiten Schritt ii) die in Schritt i) erhaltene wässrige Acrylsäure bei einem Druck von weniger als 900 mbar destillativ in eine acrylsäurereiche Phase und eine wasserreiche Phase aufgetrennt wird.

2.  Verfahren gemäß Anspruch 1, **dadurch gekennzeichnet, dass** die in Schritt i) eingesetzte wässrige Mischung aus 3-Hydroxypropionsäure und oligomerer 3-Hydroxypropionsäure von 15 bis 35 Gew.-% Wasser enthält.

3.  Verfahren gemäß Anspruch 1 oder 2, **dadurch gekennzeichnet, dass** die in Schritt i) eingesetzte wässrige Mischung aus 3-Hydroxypropionsäure und oligomerer 3-Hydroxypropionsäure von 25 bis 45 Gew.-% monomere 3-Hydroxypropionsäure enthält.

4.  Verfahren gemäß einem der Ansprüche 1 bis 3, **dadurch gekennzeichnet, dass** in Schritt i) die flüssige Phase ein hochsiedendes organisches Lösungsmittel enthält.

5.  Verfahren gemäß einem der Ansprüche 1 bis 4, **dadurch gekennzeichnet, dass** in Schritt i) die wässrige Acrylsäure mittels einer Rektifikationskolonne 2 abgetrennt wird.

6.  Verfahren gemäß einem der Ansprüche 1 bis 5, **dadurch gekennzeichnet, dass** in Schritt ii) ein Schleppmittel verwendet wird.

7.  Verfahren gemäß einem der Ansprüche 1 bis 6, **dadurch gekennzeichnet, dass** in Schritt ii) die wässrige Acrylsäure mittels einer Rektifikationskolonne 3 in eine acrylsäurereiche Phase und eine wasserreiche Phase aufgetrennt wird.

8.  Verfahren gemäß einem der Ansprüche 1 bis 7, **dadurch gekennzeichnet, dass** die Abtrennung der wässrigen Acrylsäure aus der flüssigen Phase in Schritt i) und die Auftrennung der wässrigen Acrylsäure in eine acrylsäurereiche Phase und eine wasserreiche Phase in Schritt ii) in einer Rektifikationskolonne 4 durchgeführt wird, wobei die Abtrennung der wässrigen Acrylsäure aus der flüssigen Phase unterhalb eines Seitenabzugs in der Rektifikationskolonne 4 erfolgt, die Auftrennung der wässrigen Acrylsäure in eine acrylsäurereiche Phase und eine wasserreiche Phase oberhalb des Seitenabzugs erfolgt und die acrylsäurereiche Phase am Seitenabzug flüssig entnommen wird.

9.  Verfahren gemäß Anspruch 8, **dadurch gekennzeichnet, dass** die Rektifikationskolonne 4 eine Trennwandkolonne ist, wobei sich der Zulauf zur Rektifikationskolonne 4 und der Seitenabzug der Rektifikationskolonne 4 auf unterschiedlichen Seiten der Trennwand befinden.

10. Verfahren gemäß einem der Ansprüche 1 bis 9, **dadurch gekennzeichnet, dass** die in Schritt ii) erhaltene acrylsäurereiche Phase durch Kristallisation gereinigt wird.

11. Verfahren gemäß Anspruch 10, **dadurch gekennzeichnet, dass** die Mutterlauge der Kristallisation unterhalb des Seitenabzugs in die Rektifikationskolonne 4 zurückgeführt wird.

## Claims

1.  A process for continuously dehydrating aqueous 3-hydroxypropionic acid to acrylic acid, which comprises, in a preceding step, distilling some of the water out of aqueous 3-hydroxypropionic acid, in the course of which some of the monomeric 3-hydroxypropionic acid is converted to oligomeric 3-hydroxypropionic acid with elimination of water and the mixture of monomeric 3-hydroxypropionic acid and oligomeric 3-hydroxypropionic acid thus obtained is withdrawn continuously from the bottom of the distillation, in a first step i), converting an aqueous mixture of 3-hydroxypropionic acid and oligomeric 3-hydroxypropionic acid to acrylic acid in the liquid phase and at a pressure of less than 900 mbar and separating aqueous acrylic acid from the liquid phase by distillation and, in a second step ii), separating the aqueous acrylic acid obtained in step i) at a pressure of less than 900 mbar, by distillation into an acrylic acid-rich phase and a water-rich phase.

2.  The process according to claim 1, wherein the aqueous mixture of 3-hydroxypropionic acid and oligomeric 3-hydroxypropionic acid used in step i) comprises from 15 to 35% by weight of water.

3.  The process according to claim 1 or 2, wherein the aqueous mixture of 3-hydroxypropionic acid and oligomeric 3-hydroxypropionic acid used in step i) comprises from 25 to 45% by weight of monomeric 3-hydroxypropionic acid.

4. The process according to any of claims 1 to 3, wherein the liquid phase in step i) comprises a high-boiling organic solvent.

5. The process according to any of claims 1 to 4, wherein the aqueous acrylic acid is removed in step i) by means of a rectification column 2.

6. The process according to any of claims 1 to 5, wherein an entraining agent is used in step ii).

7. The process according to any of claims 1 to 6, wherein the aqueous acrylic acid is separated in step ii), by means of a rectification column 3, into an acrylic acid-rich phase and a water-rich phase.

8. The process according to any of claims 1 to 7, wherein the removal of the aqueous acrylic acid from the liquid phase in step i) and/or separation of the aqueous acrylic acid into an acrylic acid-rich phase and a water-rich phase in step ii) is performed in a rectification column 4, with removal of the aqueous acrylic acid from the liquid phase below a side draw in the rectification column 4, separation of the aqueous acrylic acid into an acrylic acid-rich phase and a water-rich phase above the side draw and withdrawal of the acrylic acid-rich phase in liquid form in the side draw.

9. The process according to claim 8, wherein the rectification column 4 is a dividing wall column, with the feed to the rectification column 4 and the side draw from the rectification column 4 on different sides of the dividing wall.

10. The process according to any of claims 1 to 9, wherein the acrylic acid-rich phase obtained in step ii) is purified by crystallization.

11. The process according to claim 10, wherein the mother liquor from the crystallization is recycled into the rectification column 4 below the side draw.


**Revendications**

1. Procédé de déshydratation continue d'acide 3-hydroxypropionique aqueux en acide acrylique, **caractérisé en ce que** lors d'une étape en amont, une partie de l'eau est éliminée par distillation d'un acide 3-hydroxypropionique aqueux, une partie de l'acide 3-hydroxypropionique monomère se transformant en acide 3-hydroxypropionique oligomère avec clivage d'eau, et le mélange ainsi obtenu d'acide 3-hydroxypropionique monomère et d'acide 3-hydroxypropionique oligomère étant soutiré en continu du fond de la distillation, lors d'une première étape i), un mélange aqueux d'acide 3-hydroxypropionique et d'acide 3-hydroxypropionique oligomère est mis en réaction dans la phase liquide et à une pression de moins de 900 mbar pour former de l'acide acrylique, et l'acide acrylique aqueux est séparé par distillation de la phase liquide, et lors d'une deuxième étape ii), l'acide acrylique aqueux obtenu à l'étape i) est séparé par distillation à une pression de moins de 900 mbar en une phase riche en acide acrylique et une phase riche en eau.

2. Procédé selon la revendication 1, **caractérisé en ce que** le mélange aqueux d'acide 3-hydroxypropionique et d'acide 3-hydroxypropionique oligomère utilisé à l'étape i) contient 15 à 35 % en poids d'eau.

3. Procédé selon la revendication 1 ou 2, **caractérisé en ce que** le mélange aqueux d'acide 3-hydroxypropionique et d'acide 3-hydroxypropionique oligomère utilisé à l'étape i) contient 25 à 45 % en poids d'acide 3-hydroxypropionique monomère.

4. Procédé selon l'une quelconque des revendications 1 à 3, **caractérisé en ce qu'**à l'étape i), la phase liquide contient un solvant organique de point d'ébullition élevé.

5. Procédé selon l'une quelconque des revendications 1 à 4, **caractérisé en ce qu'**à l'étape i), l'acide acrylique aqueux est séparé au moyen d'une colonne de rectification 2.

6. Procédé selon l'une quelconque des revendications 1 à 5, **caractérisé en ce qu'**à l'étape ii), un agent d'entraînement est utilisé.

7. Procédé selon l'une quelconque des revendications 1 à 6, **caractérisé en ce qu'**à l'étape ii), l'acide acrylique aqueux est séparé au moyen d'une colonne de rectification 3 en une phase riche en acide acrylique et une phase riche en eau.

8. Procédé selon l'une quelconque des revendications 1 à 7, **caractérisé en ce que** la séparation de l'acide acrylique aqueux de la phase liquide à l'étape i) et la séparation de l'acide acrylique aqueux en une phase riche en acide acrylique et une phase riche en eau à l'étape ii) sont réalisées dans une colonne de rectification 4, la séparation de l'acide acrylique aqueux de la phase liquide ayant lieu en dessous d'un soutirage latéral dans la colonne de rectification 4, la séparation de l'acide acrylique aqueux en une phase riche en acide acrylique et une phase riche en eau ayant lieu au-dessus du soutirage latéral, et la phase riche en acide acrylique étant soutirée sous forme liquide par le soutirage latéral.

9. Procédé selon la revendication 8, **caractérisé en ce que** la colonne de rectification 4 est une colonne à paroi de séparation, l'alimentation de la colonne de rectification 4 et le soutirage latéral de la colonne de rectification 4 se trouvant sur des côtés différents de la paroi de séparation.

10. Procédé selon l'une quelconque des revendications 1 à 9, **caractérisé en ce que** la phase riche en acide acrylique obtenue à l'étape ii) est purifiée par cristallisation.

11. Procédé selon la revendication 10, **caractérisé en ce que** la liqueur mère de la cristallisation est recyclée en dessous du soutirage latéral dans la colonne de rectification 4.

**IN DER BESCHREIBUNG AUFGEFÜHRTE DOKUMENTE**

**In der Beschreibung aufgeführte Patentdokumente**

- WO 2012074818 A2
- US 7538247 B
- WO 2006092271 A2
- WO 2008023039 A1
- JP 2010180171 A
- EP 2565211 A1
- WO 2007106100 A1
- EP 2565212 A1
- WO 02090312 A1
- EP 0854129 A1
- EP 0616998 A1
- DE 10039025 A1
- WO 2003041832 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- WO 2011026876 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103